# EUROPÄISCHE PATENTSCHRIFT

(11) **EP 0 748 230 B1**
(45) Veröffentlichungstag und Bekanntmachung des Hinweises auf die Patenterteilung: **26.05.1999**
(21) Anmeldenummer: 95910533.9
(22) Anmeldetag: 28.02.1995
(51) Int. Cl.: A61L 2/26

(54) **STERILISATIONSBEHÄLTER**
STERILIZATION CONTAINER
CONTENEUR DE STERILISATION

(30) Priorität: 04.03.1994 DE 4407220
(43) Veröffentlichungstag der Anmeldung: 18.12.1996
(73) Patentinhaber: Maihofer, Elisabeth, 6986 Novaggio (CH)
(72) Erfinder: MAIHOFER, Willi, CH-6986 Novaggio (CH)
(74) Vertreter: von Kirschbaum, Albrecht, Dipl.-Ing.
(86) Internationale Anmeldenummer: EP9500723
(87) Internationale Veröffentlichungsnummer: WO9523617

(56) Entgegenhaltungen:
- EP-A- 0 362 612
- DE-A- 3 505 892
- DE-U- 9 404 372
- DE-U- 9 404 822

## Beschreibung

### Technisches Gebiet

Die Erfindung betrifft einen Sterilisationsbehälter, vorzugsweise für Krankenhausabfälle.

### Stand der Technik

Ein solcher Sterilisationsbehälter weist einen Kübel auf, dessen oben liegende Öffnung mit einem Deckel verschließbar ist, und er ist in erster Linie für Krankenhausfälle bestimmt, kann aber auch in anderen Bereichen Anwendung finden, wie etwa in der Arzneimittelherstellung.

Um kontaminierte Abfälle zu sterilisieren, werden diese Abfälle üblicherweise mit Dampf erhitzt. Zu diesem Zweck muß eine genügende Menge ausreichend heißen Dampfes in die Abfälle eingeleitet werden, damit zuverlässig ausgeschlossen werden kann, daß irgendein Teil des Abfalls mittels Dampf nicht intensiv und lang genug erhitzt wird, um auf jeden Fall keimfrei zu sein. Das Einleiten von Heißdampf beinhaltet ferner die Gefahr, daß entweder noch nicht ausreichend erhitzte, kontaminierte Luft oder zu sehr abgekühlter, kontaminierter Dampf in die Umgebung entweicht.

Es wurde auch schon vorgeschlagen, Mikrowellenstrahlung zur Sterilisation des Abfalls zu verwenden. Soweit dieser Anfall flüssig ist, läßt sich dieser unschwer durch Mikrowellen erhitzen, wobei jedoch Sorge dafür getragen werden muß, daß auch jene Zonen im Anfall, die infolge einer Inhomogenität der Strahlung zu wenig von dieser beaufschlagt wurden, durch Wärmeaustausch mit benachbarten Zonen ausreichend erwärmt werden.

Soweit die Abfälle aber ganz oder teilweise trocken sind, sind Mikrowellen zur Erwärmung des Abfalls ungeeignet. Aus diesem Grund ist es zweckmäßig, den Abfall soweit anzufeuchten, daß durch Verdampfung des Wassers eine so ausreichende Dampfmenge erzeugt wird, daß eine zuverlässige Sterilisierung des Anfalls erfolgt. Zum Erreichen einer ausreichenden Dampftemperatur muß der Anfall in einem Druckbehälter untergebracht werden, in welchem infolge des durch die anfängliche Verdampfung angehobenen Innendrucks die Verdampfungstemperatur ansteigt, da sich Dampf, da er gasförmig ist, durch Mikrowellen nicht weiter erwärmen läßt.

Somit setzt der Einsatz von Mikrowellenstrahlung zum Sterilisieren von Abfall die Verwendung druckfester Aufnahmebehälter für den Abfall voraus. Da solche Aufnahmebehälter aber üblicherweise sehr aufwendig sind, ist es bereits aus wirtschaftlichen Gründen nicht sinnvoll, diese Behälter auch zum Einsammeln des Abfalls vor der Sterilisierung und zum Abtransport des sterilisierten Abfalls zu verwenden.

Die Handhabung des kontaminierten Abfalls vor der Sterisilierung ist höchst gefährlich und der sterilisierte Abfall seinerseits bietet für vorhandene Krankheitskeime einen idealen Nährboden, so daß der sterilisierte Anfall am besten in einer Verpackung der Entsorgung zugeführt werden sollte, die seine Wiederinfizierung verhindert.

Aus DE-C-38 33 281 ist ein druckfester Anfallbehälter bekannt, welcher eine Sterilisierung fester Krankenhausabfälle mittels Mikrowellenstrahlung gestattet. Ferner ist aus DE-A-42 25 430 eine Vorrichtung zum Desinfizieren bzw. Sterilisieren von Müll, insbesondere von mit Keimen und Bakterien behaftetem Krankenhausmüll bekannt. Hierbei wird der Müll in einen Behälter, der mittels eines schwenkbaren Deckels mit einem Andichtungsring luftdicht abschließbar ist, eingefüllt, gegebenenfalls mit Wasser befeuchtet und anschließend mit Mikrowellen bestrahlt.

In dem Dokument DE-A-35 05 892 ist eine besonders einfache Ausführung eines verschließbaren Behälters zur Aufnahme von Laborproben, Sondermüll oder dergl. beschrieben; dieser Behälter ist als ein Einmal-Behälter konzipiert. Auch hier erfolgt die Sterilisierung durch Mikrowellenstrahlung durch die Behälter wand.

### Darstellung der Erfindung

Der Erfindung liegt daher die Aufgabe zugrunde, einen Behälter so auszubilden, daß er im Rahmen der vorstehend geschilderten Problematik in besonders einfacher, sicherer und ökonomischer Weise das Einsammeln, Sterilisieren und Entsorgen von kontaminiertem bzw.keimbelastetem Abfall gestattet.

Gemäß der Erfindung sind bei einem Sterilisationsbehälter nach Anspruch 1 zwischen dessen Kübel und dessen Decken eine druckdichte Dichtungsanordnung und eine Verschlußanordnung vorgesehen, um den Deckel auf dem Kübel entgegen der Wirkung eines Innendrucks im Kübel festzuhalten, und um ein unbeabsichtigtes Öffnen des Deckels zu verhindern. Deckel und Behälter bestehen aus einem Kunststoff, der aufgrund seiner Beschaffenheit sowohl der Druckbelastung als auch der Temperaturbelastung während des Sterilisationsvorganges zuverlässig widersteht und der für Mikrowellen durchlässig ist. Der Behälter ist wieder verwenbar.

Bevorzugte Ausführungsformen sind in den abhängigen Ansprüchen zu finden.

Ein solcher erfindungsgemäßer Sterilisationsbehälter kann mit einem Aufwand hergestellt werden, der in der Größenordnung bisheriger, einfacher Abfallbehälter für kontaminierten Abfall liegt, der aber aufgrund seines Aufbaus dazu geeignet ist, auch während der Sterilisierung mittels Mikrowellen den auftretenden Belastungen zuverlässig standzuhalten. Anschließend kann der sterilisierte Anfall im Behälter belassen werden, ohne daß dieser geöffnet zu werden braucht; soweit sich nach dem Sterilisieren ein gewisser Unterdruck einstellt, hält diesem der erfindungsgemäße Sterilisationsbehälter ebenfalls stand. Die Verschlußanordnung verhindert das unbeabsichtigte Öffnen des Behälters.

Da die Verschlußanordnung nach dem Füllen des Behälters und nach ihrem Anlegen an diesen nicht mehr gelöst zu werden braucht, bevor der sterilisierte Abfall am Bestimmungsort endgültig entsorgt wird, liegt im allgemeinen kein Grund vor, den Behälter zu öffnen, da im normalen Krankenhausbetrieb das Öffnen eines solchen Behälters nicht vorgesehen ist. Damit jedoch der Behälter nicht versehentlich geöffnet wird, kann die Verschlußanordnung vorteilhafterweise so ausgebildet werden, daß sie ohne spezielle Mittel, wie Schlüssel oder dergleichen, nicht geöffnet werden kann.

Bevorzugte Ausgestaltungen der Erfindung dienen der besseren Stapelung der noch leeren Kübel sowie der verschlossenen Behälter, der verbesserten Festigkeit des Behälters, der besseren Andichtung des Behälters sowie der besseren und zuverlässigeren Handhabung des Behälters.

Ferner ist an der Außenseite des erfindungsgemäßen Behälters gemäß einer weiteren Ausgestaltung der Erfindung eine Anordnung vorgesehen, mittels welcher die Verschlußanordnung selbst dann ständig am Kübel anbringbar ist, wenn sie nicht den Deckel am Kübel sicher festhält. Die Verschlußanordnung kann somit nicht verlorengehen. Durch ihren Zustand bzw. ihre Lage ist aber jederzeit erkennbar, daß der Kübel, selbst wenn ein Deckel aufgesetzt sein sollte, noch nicht verschlossen sein soll, so daß noch Anfall nachgefüllt werden kann.

Vorteilhafterweise ist am Behälter, und zwar am zweckmäßigsten im Deckel, vorzugsweise in dessen Mitte, ein Bereich mit verringerter Wandstärke vorgesehen, der von einer geeigneten Versteifung, wie etwa einer Ringsicke, umgeben ist, wodurch verhindert ist, daß es zu einem übermäßigen Auswölben infolge eines Innendrucks im Behälter kommt. Die Wandstärke dieses Bereichs ist so gering, daß er mittels einer entsprechend bemessenen Injektionsnadel durchstoßen werden kann, mittels welcher dann Wasser oder eine sonstige Flüssigkeit zum Anfeuchten des Anfalls vor der Mikrowellenbehandlung in den Behälter eingespritztwerden kann.

Nach dem Einspritzen kann entweder die Injektionsnadel dichtend in der von ihr geschaffenen Perforierung verbleiben, nachdem sie selbst verschlossen wurde, oder es ist möglich, durch die Perforierungsöffnung einen Dichtungsstopfen einzuführen, der sich an seinem im Behälter liegenden Ende pilzförmig ausdehnt und somit durch den Innendruck im Behälter dichtend angepreßt wird. Der sich außerhalb des Behälters befindende Anschnitt des Dichtungsstopfens kann soweit verschweißt, gestaucht oder sonstwie verformt werden, daß er bei Auftreten eines Unterdrucks im Behälter nicht in diesen hineinrutscht.

Der erfindungsgemäße Sterilisationsbehälter weist vorzugsweise einen kreisförmigen Querschnitt auf, kann jedoch auch jeden anderen geeigneten Querschnitt annehmen. Die Hauptabmessungen des erfindungsgemäßen Sterilisationsbehälters entsprechen vorzugsweise denjenigen einer kleinen Haushalts-Mülltonne.

Die im folgenden verwendeten, räumlichen Bezugsbegriffe, wie "oben", "unten", "über", "unter" usw. beziehen sich auf den erfindungsgemäßen, auf einer ebenen Fläche aufrecht aufgestellten Sterilisationsbehälter, dessen Einfüllöffnung oben liegt und der sich somit in seiner Gebrauchslage befindet.

### Kurze Beschreibung der Zeichnungen

Nachfolgend wird die Erfindung anhand von bevorzugten Ausführungsformen unter Bezugnahme auf die beigefügten, schematischen Zeichnungen im einzelnen erläutert. Es zeigen:
- Fig.1: einen Teil einer Schnittansicht eines Ausführungsbeispiels des erfindungsgemäßen Behälters mit aufgesetztem Deckel;
- Fig.2: eine Teil-Draufsicht auf den Kübel des Behälters der Fig.1;
- Fig.3: eine Teil-Draufsicht auf den Deckel des Behälters der Fig.1;
- Fig.4: eine Teil-Draufsicht auf einen in Fig.1 verwendeten Verschlußring;
- Fig.5: eine Ansicht aus der Richtung V in Fig. 4;
- Fig.6: eine Schnittansicht entlang der Linie VI-VI in Fig. 4;
- Fig.7: eine Draufsicht auf ein Spannschloß des Verschlußrings;
- Fig.8: vergrößert eine Einzelheit VIII in Fig.1, und
- Fig.9: eine Fig.8 ähnliche Darstellung einer Ausführungsvariante

### Bester Weg zur Ausführung der Erfindung

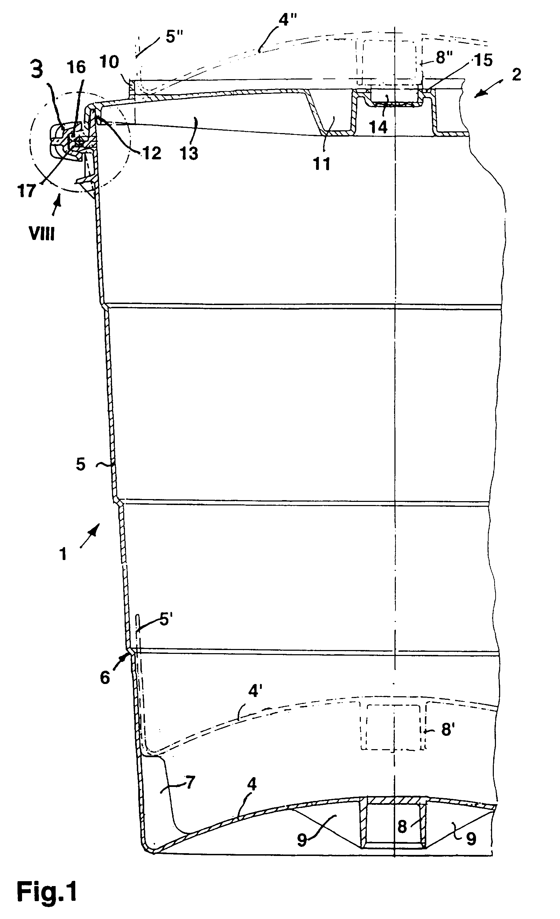

In Fig.1 ist in einem Teil einer Schnittansicht ein Kübel 1 dargestellt, dessen Seitenwand 5 aus sich nach unten verjüngenden Kegelstumpfabschnitten besteht, die unter Bildung von Ansätzen 6 nach unten in jeweils einen kleineren Anschnitt übergehen. Der Kübel 1 weist einen nach oben gewölbten Boden 4 auf. Am Übergang zwischen Boden 4 und Seitenwand 5 ist im Inneren des Kübels 1 ein Kranz von aufrechten Versteifungsrippen 7 ausgebildet, die jeweils eine im wesentlichen ebene Oberkante aufweisen.

In Fig.1 ist gestrichelt der Boden 4' und die Seitenwand 5' eines zweiten Kübels angedeutet, der in den dargestellten Kübel stapelnd eingesetzt ist. Wie erkennbar, sitzt der Innenkübel mit seiner unteren Umfangskante am Außenumfang des Bodens 4' bzw. der Unterkante der Seitenwand 5' auf der Oberseite aller Boden-Versteifungsrippen 7 auf.

In der Mitte des Bodens 4 erstreckt sich nach unten ein Rohrstutzen 8, der im wesentlichen zylindermantelförmig ausgebildet ist und an seinem unteren Ende von innen nach außen und unten abgeschrägt ist. Wie aus Fig.1 zu ersehen ist, liegt bei dem nicht durch einen Innendruck beaufschlagten Kübel 1 die Unterkante des Rohrstutzens 8 über der Fläche, auf der der Kübel 1 abgestellt ist. Zwischen der Außenseite des Rohrstutzens 8 und der Unterseite des Bodens 4 ist ein Kranz von Stutzen-Versteifungsrippen 9 ausgebildet.

Bei dem gestrichelt angedeuteten, in den Kübel 1 eingesetzten, zweiten Kübel ist dessen Rohrstutzen 8' zu erkennen, welcher zum Boden 4 des ersten Kübels 1 einen beträchtlichen Anstand aufweist, der durch die Höhe der Boden-Versteifungsrippen bestimmt wird. Diese Höhe ist so festgelegt, daß alle an der Außenseite der Seitenwand 5' angeordneten Vorsprünge, die in Verbindung mit Fig.8 näher beschrieben werden, an einem stapelnd in den Kübel 1 eingesetzten, zweiten Kübel über der Oberkante der Seitenwand 5 des Kübels 1 liegen und hierbei weder beschädigt werden noch behindern. Im übrigen sorgt die abgestufte Ausbildung der Seitenwand 5 dafür, daß die stapelnd ineinandergeschobenen Kübel 1 klemmungsfrei ineinander sitzen.

Die Oberseite des Kübels 1 ist durch einen Deckel 2 verschlossen, der nach oben ausgewölbt ist. Nahe dem Rand des Deckels 2 ist an seiner Oberseite ein Außen-Ringsteg 10 angeformt, der als kurzer Zylindermantel ausgebildet ist. Der Durchmesser des Außen-Ringstegs 10 ist so bemessen, daß er ein wenig größer ist als der Außenumfang des Kübels 1 an seiner Unterseite.

Wie in der Zeichnung gestrichelt angedeutet, kann auf den Deckel 2 des verschlossenen Behälters ein zweiter Behälter aufgesetzt werden, von dem das untere Ende dessen Seitenwand 5'' und dessen Boden 4'' angedeutet sind. Hierbei sitzt die Unterkante dieses oberen Kübels innerhalb des Außen-Ringstegs 10, wodurch das seitliche Abrutschen des oberen Kübels verhindert ist.

In der Mitte des Deckels ist eine nach unten eingebuchtete Ringsicke 11 ausgebildet. Nahe dem Außenumfang des Deckels 2 ist ein sich nach unten erstreckender Innen-Ringsteg 12 ausgebildet, dessen Innenflanke kreiszylindrisch und dessen Außenflanke als ein sich nach unten verjüngender Kegelmantel ausgebildet ist. Zwischen der Innenflanke des Innen-Ringstegs 12 und der Außenflanke der Ringsicke 11 ist eine Gruppe strahlenartig angeordneter Deckel-Versteifungsrippen 13 angeordnet.

Der von der Ringsicke 11 umgebene, mittlere Bereich des Decksl 2 weist eine zur Ringsicke 11 und zum Deckel 2 konzentrische Ringnut 15 auf, die so bemessen ist, daß in ihr die Unterkante des Rohrstutzens 8'' eines oberen, stapelnd aufgesetzten Kübels absetzbar ist, wenn dieser Kübel unter Innendruck steht, durch den der Boden 4'' und damit auch der Rohrstutzen 8'' ein wenig nach außen gedrückt wird. Im Bereich innerhalb der Ringnut 15 ist eine flache Vertiefung 14 ausgebildet, deren Boden dünnwandiger als die übrigen Teile des Deckels 2 und des Kübels 1 ausgebildet ist.

An der Unterseite des Deckels 2 ist ein Deckel-Außenflansch 16 ausgebildet, der auf einem Kübel-Außenflansch 17 aufsitzt, der an der Außenseite der Seitenwand 5 des Kübels 1 etwas unter deren Oberkante ausgebildet ist. Von außen her ist ein Verschlußring 3 über die beiden Außenflansche 16, 17 klemmend aufgeschoben.

Es wird nun auf Fig.8 übergegangen, in der die Einzelheit VIII in Fig.1 vergrößert dargestellt ist, und anhand der die Einzelheit VIII näher erläutert wird . Die Elemente in Fig.8, die schon im Zusammenhang mit Fig.1 beschrieben sind, werden, soweit nicht für die Erläuterung zweckmäßig und erforderlich, nicht mehr wiederholt.

Der Deckel-Außenflansch 16 und der Kübel-Außenflansch 17 weisen jeweils einander zugewandt eine ebene Ringfläche auf, in die eine deckelseitige Ringnut 19 mit im wesentlichen halbkreisförmigem Querschnitt bzw. eine kübelseitige Ringnut 18 mit insgesamt etwa quadratischem Querschnitt und gleicher Breite eingebracht sind.

Wie zu erkennen ist, sind die Seitenflanken, mit denen die deckelseitige Ringnut 19 in die untere Fläche des Deckel-Außenflansches 16 übergeht, stark abgerundet. Die beiden Ringnuten 18, 19 bilden einen Ringkanal, in den ein Dichtungsring 20 aus flexiblem Material eingelegt ist.

Die beiden Abrundungen beiderseits der deckelseitigen Ringnut 19 bilden beiderseits des Dichtungsringes 20 schmale Zwickel, in welche das Material des Dichtungsringes 20, wenn dieser durch Zusammenpressen der beiden Außenflansche 16, 17 zusammengedrückt wird, ausweichen kann. Somit ist die Höhe des durch die Ringnuten 18, 19 gebildeten Ringkanals ein wenig geringer als die Höhe des unbelasteten Dichtungsringes 20, während die Querschnittsfläche des erwähnten Ringkanals 18, 19 etwa der des Dichtungringes 20 entspricht oder gegebenenfalls größer ist.

An der freien Außenkante des Kübel-Außenflansches 17 ist ein nach oben vorspringender, sich nach oben und außen verjüngender Ringvorsprung 21 ausgebildet, der passend in einer komplementären Vertiefung 16b am Außenrand des Deckel-Außenflansches 16 aufgenommen ist. Die Innenkante des Kübel-Außenflansches 17 geht mit einem Radius in einen sich nach oben verjüngenden, kegeligen Anschnitt 5b der Außenfläche der Seitenwand 5 über, die sich weitr nach oben fortsetzt und in einer Ausrundung 5a an der Einfüllöffnung endet.

Die Außenwand 22 des Deckels 2 ist im unteren Anschnitt 22a, an dem der Deckel-Außenflansch 16 angesetzt ist, komplementär zur Abschrägung 5b der Kübel-Seitenwand 5 ausgebildet. Oberhalb des komplementären Abschnitts 22a ist zwischen dem inneren Ringsteg 12 und der Außenwand 22 des Deckels 2 eine nach unten offene Dichtungsnut 2a gebildet, deren Grund abgerundet ist und deren Seitenflanken nach unten divergieren. Wie zu erkennen ist, ist der Radius der Ausrundung des Nutgrundes der Dichtungsnut 2a größer als der Radius der Ausrundung 5a der Oberkante der Seitenwand 5. Außerdem berührt der obere Abschnitt der Seitenwand 5 in unbelastetem Zustand nicht die Flanken der Dichtungsnut zwischen der Deckel-Außenwand 22 und dem Ringsteg 12.

Die obere Fläche des Deckel-Außenflansches 16 und die untere Fläche des Kübel-Außenflansches 17 konvergieren nach außen; um diese beiden Außenflansche 16, 17 fest zusammenzuhalten, ist von außen her ein Verschlußring 3 aufgeschoben, der eine den beiden Außenflanschen 16, 17 zugewandte, zu deren Außenflächen komplementär ausgebildete Innennut 23 aufweist und auf diese aufklemmbar ist. Weitere Einzelheiten des Verschlußringes 3 werden weiter unten noch beschrieben.

Wenn der Ring 3 von außen her auf die beiden Außenflansche 16, 17 aufgeschoben oder aufgedrückt ist, dann findet eine Dichtung statt:
- am Nutgrund der Dichtungsnut zwischen Deckel-Außenwand 22 und Innen-Ringsteg 12, wo die Oberkante (Ausrundung 5a) der Seitenwand 5 dichtend aufsitzt,
- an den komplementären konischen Flächen (den Dichtungsbereichen 22a, 5b) von Deckel-Außenwand 22 und Seitenwand 5, wo diese beiden mit einem Kegelsitz aufeinandersitzen,
- an den horizontalen Anlageflächen der beiden Außenflansche 16,17,
- zwischen Ringvorsprung 21 und komplementärer Ausbildung (16b) des Deckel-Außenflansches 16, und
- durch den Dichtungsring 20.

Hierbei ist durch die beiden seitlichen Zwickel, die an den durch die Ringnuten 18, 19 gebildeten Ringkanal angrenzen, der den Dichtungsring 20 aufnimmt, dafür gesorgt, daß, auch wenn der Dichtungsring 20 ganz zusammengedrückt ist, die beiden Außenflansche 16, 17 satt und dicht aneinander anliegen.

Bei einem hohen Innendruck im Kübel 1 wird möglicherweise der obere Anschnitt der Seitenwand 5, der sich innerhalb der Dichtungsnut befindet, nach außen ausgewölbt. Hierdurch rollt sich aber lediglich der Außenradius der Oberkante der Seitenwand 5 am Nutgrund der Dichtungsnut ab. Die Dichtung selbst wird dadurch nicht beeinträchtigt.

Um zu verhindern, daß sich bei Belastung der Kübel-Außenflansch 17 auch verformt oder nachgibt, ist dieser, wie in Fig.8 durch eine gestrichelte Linie gezeigt, durch einen Kranz von Versteifungsrippen zur Außenseite der Seitenwand 5 des Kübels 1 hin abgestützt.

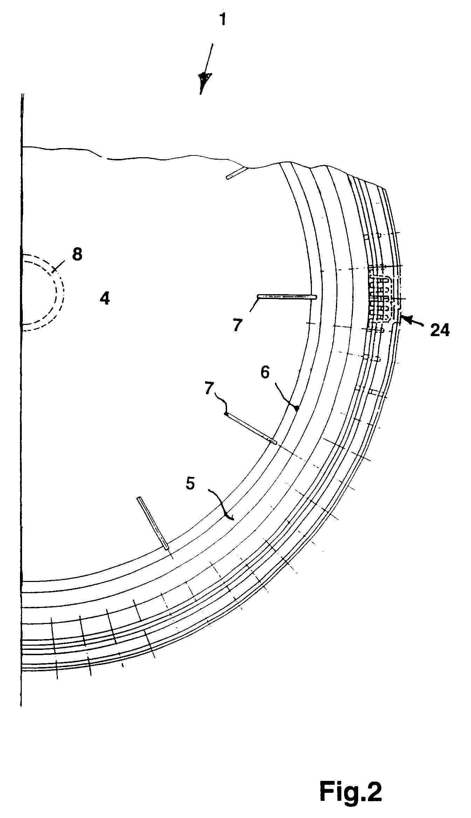

Über den Außenumfang der Seitenwand 5 des Kübels 1 verteilt sind vier Konsolen 24 angeordnet, die in Form kurzer, leicht nach außen und unten geneigter Flächenabschnitte ausgebildet sind, welche jeweils durch Konsolen-Versteifungsrippen 25 nach unten und zur Seitenwand 5 des Kübels 1 hin abgestützt sind. Um den Kübel-Außenflansch 17 noch besser abzustützen, sind zwischen dessen Unterseite und der Oberseite der Konsole 24 weitere Versteifungsrippen 26 angeordnet. Die Lage der Konsole 24 ist insbesondere aus Fig.2 zu ersehen.

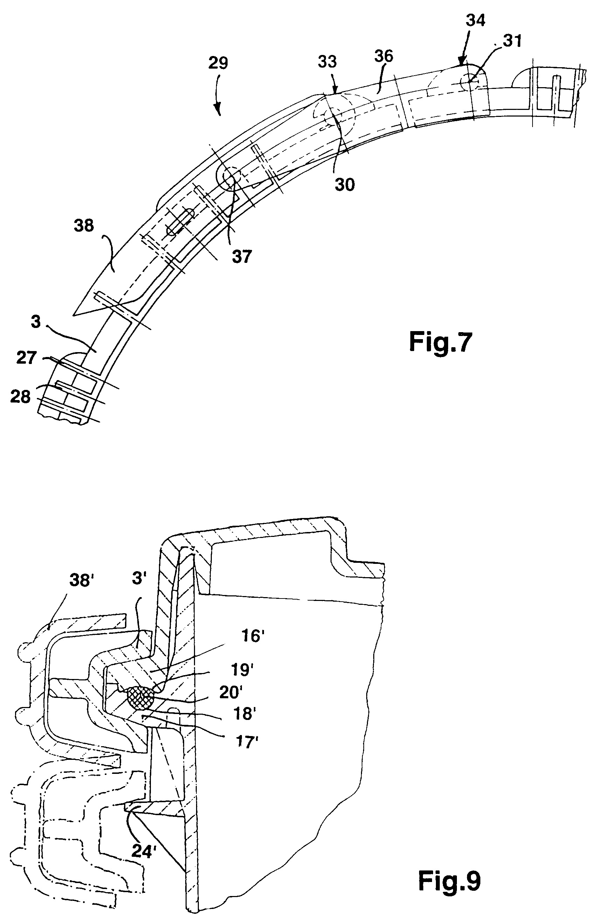

Der Verschlußring 3, der sich bei geschlossenem Behälter in der in Fig.8 gezeigten Lage befindet, kann bei leerem Behälter über die Konsolen 24 gehängt werden, wie strichpunktiert in Fig.9 gezeigt ist. Somit ist bei offenem Behälter und frei zugänglichem Innenraum des Kübels 1 der Ring 3 unverlierbar an diesem befestigt.

Bei geschlossenem Behälter (der Ring befindet sich in der Lage der Fig.8) dienen die Konsolen 24 als Handgriffe zum Anheben des Behälters. Hierdurch ist verhindert, daß der volle und somit gegebenenfalls schwere Behälter am Ring 3 erfaßt und angehoben wird, wodurch unter Umständen die Abdichtung beeinträchtigt und eine Beschädigung verursacht werden könnte.

Bei der Variante der Fig.9 ist, wie ersichtlich, die kübelseitige Ringnut 18' als über ihre volle Breite ausgerundete Nut ausgebildet; die deckelseitige Ringnut 19' ist als eine flache Vertiefung mit einem sehr viel größeren Radius ausgeführt, so daß die offene Seite der deckelseitigen Ringnut 19' viel breiter ist als die kübelseitige Ringnut 18'. Aus diesem Grund entstehen zwei sehr reichlich bemessene Randzwickel beiderseits der Ringdichtung 20', in welche hinein diese beim Festspannen des Deckels und der dadurch bedingten Verformung ausweichen kann, so daß der Dichtungsring 20' nicht das satte Aneinanderliegen der beiden Außenflansche 16', 17' behindert.

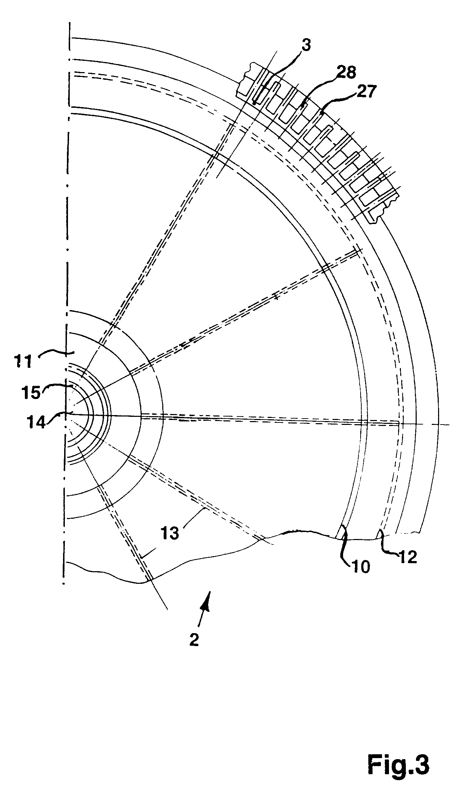

In Fig.2 ist eine Draufsicht von oben her in den Kübel 1 wiedergegeben; die Boden-Versteifungsrippen 7 sind zu erkennen; eine von vier Konsolen 24 ist gestrichelt angedeutet. In Fig.2 ist die große Anzahl von Versteifungsrippen entnehmbar, die den Außenflansch des Kübels 1 zu dessen Seitenwand 5 hin abstützen und für eine dauerhafte und steife Ausbildung sorgen. In Fig.3 ist die Draufsicht auf den Deckel 2 wiedergegeben, wobei ein weggebrochenes Stück des Ringes 3 zu sehen ist.

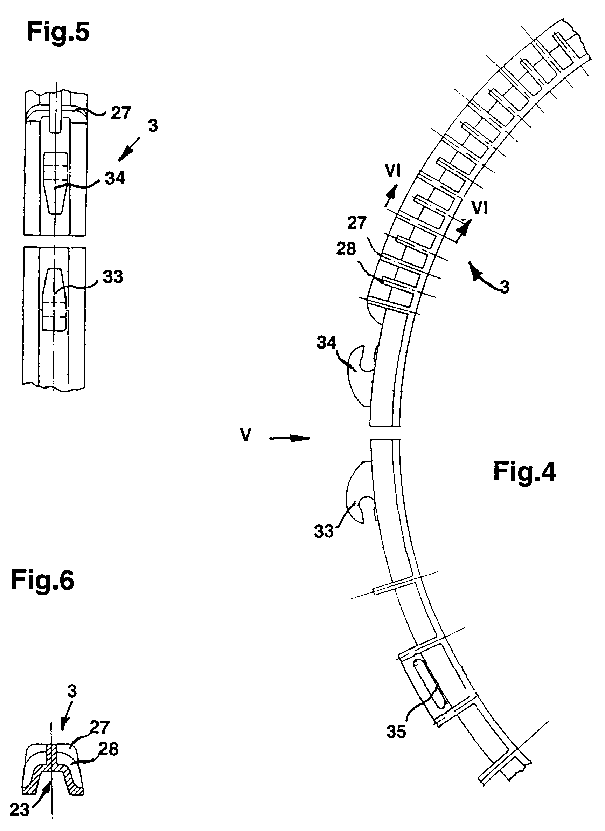

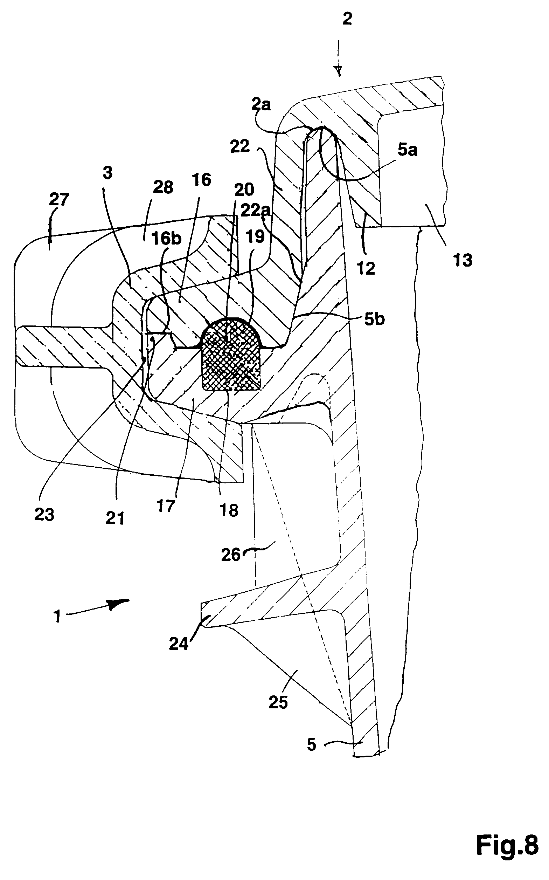

Der Querschnitt des Verschlußringes 3 ist am besten Fig.6 zu entnehmen. Wie Fig.4 und 6 zu entnehmen ist, sind an der Außenseite des Ringes 3 alternierend kurze Versteifungsrrippen 28 und lange Versteifungsrippen 27 ausgebildet.Hierdurch wird der Ring insgesamt in radialer Richtung ein wenig flexibel, ist jedoch in axialer Richtung außerordentlich steif, so daß die Innenut 23 stets unverformt bleibt.

Das Spannschloß 29 ist aus den Fig. 4, 5 und 7 zu entnehmen. Der Verschlußring 3 ist an einer Stelle radial geschlitzt; beiderseits dieses Schlitzes sind zwei voneinander weg weisende, nach außen abstehende Haken 33, 34 angebracht. Ferner ist auf der Seite des Schlitzes, auf der der Haken 33 angeordnet ist, mit Anstand von diesem ein Steg mit einem sich in Umfangsrichtung des Ringes erstreckenden Langloch 35 ausgebildet.

Ein Spannhebel 38 (Fig.7) ist mit einem Druckbolzen 30 in den Haken 33 eingeschnappt. Ein längliches Zugglied 36, das auch aus zwei übereinanderliegenden, den Spannhebel 38 einschließenden, flachen Zugelementen gebildet sein kann, ist über einem Zugbolzen 31 schwenkbar im Zughaken 34 gehaltert. Das andere Ende des Zuggliedes ist mittels eines Koppelbolzens 37 schwenkbar am Spannhebel 38 angebracht

Wird der Spannhebel aus der in Fig.7 dargestellten Lage mit seinem freien Ende in horizontaler Richtung vom Ring 3 weggeklappt, dann verkürzt sich die Strecke zwischem dem Zugbolzen 31 und dem Koppelbolzen 37, und die beiden Bolzen 30, 31 und damit auch die beiden Haken 33, 34 werden bei diesem Vorgang auseinandergedrückt. Der Ring 3 wird dadurch aufgeweitet und kann von den beiden Außenflanschen 16, 17 abgenommen bzw. über diese geschoben werden. Wird der Spannhebel 38 dann wieder zurückgedrückt, dann wird der Ring 3 wieder auf sein Paßmaß verkürzt. Die mögliche Längung des Ringes 3 in Umfangsrichtung gleicht hierbei aufgetretene Toleranzen aus.

Der Spannhebel 38 weist außerdem eine (in der Zeichnung nicht dargestellte) Bohrung auf, die mit dem Langloch 35 zur Deckung gebracht werden kann, so daß ein Schloß, Splint oder dergleichen durchgesteckt werden kann, um den Spannhebel 38 in seiner geschlossenen Lage festzulegen.

In Fig.9 ist der Spannhebel 38' im Schnitt gezeigt; dieser ist, wie der Spannhebel 38 der Fig.8, bevorzugt aus einem für Mikrowellen durchlässigen, zähen Kunststoff gebildet. Fig.5 ist die Ansicht V in Fig.4 und zeigt die Draufsicht auf die beiden Haken 33, 34 bei abgenommenem Spannhebel 38. In Fig.9 ist der Spannhebel 38' im Schnitt dargestellt. Alle beschriebenen Bohrungen und Bolzen verlaufen parallel zur Achse des Ringes 3, also gemäß Fig.1 in vertikaler Richtung. Bezüglich der genauen Ausbildung der einzelnen Elemente wird ausdrücklich auf die Zeichnung verwiesen.

Zur Bereitstellung der gezeigten Sterilisationsbehälter werden die Kübel 1 ineinander gestapelt, wie dies in Fig.1 gezeigt ist. Hierbei ist bei jedem Kübel der Ring 3 über die Konsolen 24 bzw. 24' geschlossen, wie dies in Fig.9 gezeigt ist. Das Spannschloß 29 des Ringes 3 ist ebenfalls geschlossen. Der Ring 3 ist somit unverlierbar am Kübel 1 gehalten, stört aber das Stapeln wegen der entsprechend hoch ausgebildeten BodenVersteifungsrippen nicht. Die Deckel 2 sind separat abgelegt, wobei die Deckel-Versteifungsrippen 13 des einen Deckels auf der Oberkante des Außen-Ringstegs 10 des darüber oder darunter liegenden Deckels 2 aufsitzen.

Nunmehr wird ein Kübel dem Stapel entnommen und beispielsweise mit Krankenhausabfall gefüllt. Spätestens, wenn er voll ist, wird ein Deckel 2 dem Deckel-Stapel entnommen, und in die von der Seitenwand 5 des Kübels 1 gebildete Öffnung so eingesetzt, daß die Dichtungsnut die obere Kante der Seitenwand 5 umfaßt. Der Ring 3 wird dann mittels des Spannhebels 38 auseinandergespreizt, von den Konsolen 24 bzw. 24' abgezogen und über die beiden Außenflansche 16, 17 des Deckels 2 bzw. des Kübels 1 geschoben. Anschließend wird der Spannhebel 38 wieder gegen die Außenfläche des Ringes 3 geklappt, wodurch der Ring festgezogen ist.

Dadurch ist der Inhalt des Kübels gegenüber der Umgebung hermetisch und mehrfach abgedichtet. Vor der Sterilisierung kann mittels einer Hohlnadel der dünnwandige Boden der flachen Vertiefung 14 des Deckels 2 durchstoßen und die gewünschte Menge an Flüssigkeit, beispielsweise Wasser, in das Innere des Behälters eingespritzt werden; es ist aber auch möglich, eine bestimmte Flüssigkeitsmenge vor Aufsetzen des Deckels 2 in den Kübel zu gießen.

Nach Andichten der Einstichstelle auf irgendeine bekannte Weise wird der mit angefeuchtetem Abfall gefüllte Sterilisationsbehälter in eine Mikrowellenkammer eingebracht und dort der Mikrowellenbestrahlung ausgesetzt. Durch diese wird die Flüssigkeit im Inneren des Kübels 1 zum Verdampfen gebracht, wobei sich nach einiger Zeit ein Druck-Temperatur-Gleichgewicht einstellt. Durch die Dampfentwicklung wird der gezeigte Behälter geringfügig aufgebläht; die an allen Gefährdungsstellen angeordneten Versteifungsrippen sorgen dafür, daß sowohl die Abdichtung als auch die Unversehrtheit des Behälters erhalten bleibt.

Die Mikrowellen können die Wandungen des Behälters unbehindert durchdringen. Nach abgeschlossener Erhitzung und somit Sterilisation des Behälterinhalts wird der Behälter zur Entsorgung bereitgestellt, wobei sich in seinem Inneren ein gewisser Unterdruck einstellen kann. Auch in diesem Fall sorgen die Versteifungsrippen sowie die Ausbildung der Dichtungsanordnung dafür, daß Andichtung und Behälter unversehrt bleiben.

Nach Durchführen der Entsorgung und gegebenenfalls nach Reinigung des Behälters können dessen Kübel und Deckel zur erneuten Verwendung gestapelt werden.

## Patentansprüche

1. Wiederverwendbarer Sterilisationsbehälter, vorzugsweise für Krankenhausabfälle, mit
einem Kübel aus für Mikrowellen durchlässigem Material (1) mit einer von der Oberkante seiner Seitenwand begrenzten, seinem Boden gegenüberliegenden Einfüllöffnung,
einem Deckel (2) zum Andecken der Einfüllöffnung, der bevorzugt aus dem gleichen, für Mikrowellen durchlässigen Material besteht wie der Kübel (1), und
einer Dichtungsanordnung mit Dichtungsring (20) zwischen dem Kübel (1) und dem Deckel (2), wobei
zur Gewährleistung einer sicheren und zuverlässigen, druckdichten Andichtung zwischen dem Kübel (1) und dem Deckel (2) die Dichtungsanordnung außer dem Dichtungsring (20), der in seiner Querschnittsform entsprechend ausgebildeten Nuten (18, 19) im Kübel (1) und im Deckel (2) untergebracht ist, zusätzliche Dichtungsbereiche (21, 16b; 5b, 22a; 5a, 2a) am Kübel (1) und am Deckel (2) aufweist, und
eine lösbare, gegebenenfalls in der geschlossenen Lage sicherbare Verschlußanordnung (3, 29) zum Festhalten des Deckels (2) auf dem Kübel (1) entgegen der Wirkung eines Innendrucks im Kübel (1) vorgesehen ist, und
der Kübel (1), gegebenenfalls auch der Deckel (2), aus einem zähen, auch über die Siedetemperatur von Wasser hinaus noch dehnungsbeständigen Kunststoff besteht.

2. Behälter nach Anspruch 1, **dadurch gekennzeichnet**, daß die Seitenwand (5) des Kübels (1) sich insgesamt von der Einfüllöffnung bis zum Boden (4) hin verjüngt und in einen anderen Kübel zum Stapeln der Kübel einschiebbar ist.

3. Behälter nach Anspruch 1, **dadurch gekennzeichnet**, daß die Innen-Umfangskante zwischen Seitenwand (5) und Boden (4) des Kübels (1) durch einen Kranz von Boden-Versteifungsrippen (7) ausgesteift ist, deren Oberkanten eine Auflage für den Außenrand des Bodens (4') eines zweiten, eingesetzten Kübels bilden und daß der Abstand aller von der Außenseite der Seitenwand (5) nach außen abstehenden Vorsprünge (24, 17) zur Oberkante der Seitenwand (5) kleiner ist als die Höhe der Boden-Versteifungsrippen (7).

4. Behälter nach einem der Ansprüche 2 oder 3, **dadurch gekennzeichnet**, daß die Außenwand (5) aus einer Reihe übereinanderliegender, gegeneinander abgesetzter Kegelstumpfmäntel gebildet ist.

5. Behälter nach einem der Ansprüche 1 bis 4, **dadurch gekennzeichnet**, daß an der Unterseite des zur Kübel-Innenseite hin gewölbten Bodens (4) ein mittiger Boden-Rohrstutzen (8) ausgebildet ist, der bei nicht druckbeaufschlagtem Kübel (1) nicht bis zur Höhe der Unterkante der Seitenwand (5) hinunterreicht.

6. Behälter nach einem der Ansprüche 1 bis 5, **dadurch gekennzeichnet**, daß an der Oberseite des vorzugsweise nach oben gewölbten Deckels (2) ein nach oben abstehender, vorzugsweise durchgehender Außen-Ringsteg (10) ausgebildet ist, der zur Unterkante des Kübels (1) komplementär ist und beim Stapeln verschlossener Behälter die Kübel-Unterkante aufnimmt.

7. Behälter nach einem der Ansprüche 1 bis 6, **dadurch gekennzeichnet**, daß nahe dem Außenumfang des Deckels (2) ein nach unten abstehender Innen-Ringsteg (12) ausgebildet ist, der passend an dem Innenumfang der Seitenwand (5) des Kübels (1) anliegt.

8. Behälter nach einem der Ansprüche 1 bis 7, **dadurch gekennzeichnet**, daß in der Mitte des Deckels (2) eine zur Innenseite des Behälters hin eingebuchtete Ringsicke (11) ausgebildet ist, wobei die Mittelzone des nicht eingebuchteten Kernbereichs der Ringsicke (11) als flache Vertiefung (14) ausgebildet ist und eine gegenüber der sonstigen Wandstärke des Deckels (2) verringerte Wandstärke aufweist.

9. Behälter nach Anspruche 8, **dadurch gekennzeichnet**, daß in der Randzone des nicht eingebuchteten Kernbereichs der Ringsicke (11) eine Ringnut (15) angeordnet ist, die komplementär zur Unterkante des Boden-Rohrstutzens (8) ausgebildet ist.

10. Behälter nach einem der Ansprüche 1 bis 9, **dadurch gekennzeichnet**, daß an oder nahe der Oberkante des Kübels (1) sowie am Außenrand des Deckels jeweils ein Außenflansch (16,17) ausgebildet ist, und daß zwischen den Außenflanschen (16,17) mindestens ein flexibler Dichtungsring (20) angeordnet ist, zu dessen Aufnahme in mindestens einem der Außenflansche (16,17) eine einen Ringkanal bildende Ringnut (18,19) ausgebildet ist, wobei die Höhe des Ringkanales (18,19) kleiner ist als die Höhe des unbelasteten Dichtungsringes (20), und die Querschnittsfläche des Ringkanales (18,19) mindestens ebenso groß ist, wie die des Dichtungsringes (20).

11. Behälter nach Anspruch 10, **dadurch gekennzeichnet**, daß außerhalb des Ringkanales (18,19) einer der Außenflansche (16,17) vorzugsweise der Kübel-Außenflansch (17), einen Ringvorsprung (21) aufweist und der andere Außenflansch, vorzugsweise der Deckel-Außenflansch (16) eine zu dem Ringvorsprung (21) komplementäre Aussparung aufweist.

12. Behälter nach einem der Ansprüche 10 oder 11, **dadurch gekennzeichnet**, daß die Verschlußanordnung zum Festhalten des Deckels (2) auf dem Kübel (1) als Einrichtung (3, 29) zum Zusammenspannen der beiden Außenflansche (16,17) ausgebildet ist.

13. Behälter nach Anspruch 12, **dadurch gekennzeichnet**, daß die nach unten weisende Fläche des Kübel-Außenflansches (17) und die nach oben weisende Fläche des Deckel-Außenflansches (16) nach außen zueinander konvergieren und insbesondere gemeinsam einen Querschnitt in Form eines gleichschenkligen Trapezes aufweisen, und daß eine Klemmeinrichtung mit mindestens einem Klemmelement (3) vorgesehen ist, das einen komplementären Querschnitt aufweist und von außen her wie eine Spange über die beiden Außenflansche (16,17) aufschiebbar ist.

14. Behälter nach Anspruch 13, **dadurch gekennzeichnet**, daß die Neigungswinkel der nach unten weisenden Fläche des Kübel-Außenflansches (17) und der nach oben weisenden Fläche des Deckel-Außenflansches (16) jeweils gegenüber der Aufschieberichtung des mindestens einen Klemmelementes (3) vorzugsweise gegenüber der Horizontalen, jeweils den Selbsthemmungswinkel übersteigen.

15. Behälter nach Anspruch 13 oder 14, **dadurch gekennzeichnet**, daß das Klemmelement als ein die beiden Außenflansche (16,17) von außen her umgreifender Ringkörper (3) ausgebildet ist, der bevorzugt als einteiliger, federnder Ring (3) aus Kunststoff, vorzugsweise dem gleichen Kunststoff, aus dem der Kübel (1) und/oder der Deckel (2) besteht, ausgebildet ist, daß der Ring (3) eine zur Mitte hin weisende Innennut (23) aufweist, deren Querschnitt demjenigen der beiden Außenflansche (16,17) entspricht, daß der Ring (3) an mindestens einer Stelle einen Radialschlitz aufweist, und daß der Radialschlitz durch eine Spannvorrichtung (29) überbrückt ist.

16. Behälter nach Anspruch 15, **dadurch gekennzeichnet**, daß die Spannvorrichtung (29) einen Spannhebel (38) aufweist, welcher mit seinem einen Ende am Ring (3) neben der einen Seite des Radialschlitzes um eine zur Ringachse parallele Schwenkachse schwenkbar gelagert ist, und welcher über ein beidseitig gelenkig angekoppeltes Zugglied (36) mit einer Stelle des Ringes (3) an der anderen Seite des Radialschlitzes verbunden ist.

17. Behälter nach einem der Ansprüche 14 oder 15, **dadurch gekennzeichnet**, daß der Ring (3) einen im wesentlichen Y-förmigen Querschnitt aufweist, wobei die Innenflanken der beiden Schenkel des Y die Innennut (23) begrenzen, und daß zwischen den Außenflanken der beiden Schenkel des Y und der entsprechenden Seite des Längsbalkens des Y jeweils Ring-Versteifungsrippen (27,28) ausgebildet sind.

18. Behälter nach einem der Ansprüche 15 bis 17, **dadurch gekennzeichnet**, daß zur Anlage des Ringes (3) bei abgenomenem Deckel (2) an der Außenseite der Seitenwand (5) des Kübels (1) unterhalb des Kübel-Außenflansches (17) eine Ringauflage (24), vorzugsweise aus vier, über den Außenumfang der Seitenwand (5) verteilten Konsolen (24) gebildet ist, wobei der von den Konsolen (24) beschriebene Außenumfang kleiner ist als der Außendurchmesser der Außenflansche (16,17) und größer als der Innendurchmesser des Ringes (3) mit geschlossener Spannvorrichtung (29), so daß der geschlossene Ring (3) die Außenkanten der Konsolen (24) lose, aber unverlierbar umschließt und die Konsolen (24) vorzugsweise durch ihre nach unten und außen leicht geneigte Anordnung als Handgriffe ausgebildet sind.

19. Behälter nach einem der Ansprüche 7 bis 18, **dadurch gekennzeichnet**, daß die Seitenwand (5) des Kübels (1) über den Kübel-Außenflansch (17) hinaus nach oben verlängert ist, und daß der Deckel (2) zwischen dem Innen-Ringsteg (12) und seiner die Oberkante des Kübels (1) außen umgreifenden, sich bis zum Deckel-Außenflansch (16) erstreckenden Deckel-Außenwand (22) eine nach unten offene Dichtungsnut aufweist, in welche sich die Oberkante der Seitenwand (5) des Kübels (1) erstreckt, wobei sich vorzugsweise der Nutgrund der Dichtungsnut nach oben hin verjüngt und in einer Ausrundung endet, die Oberkante der Seitenwand (5) des Kübels (1) eine Abrundung aufweist, die auf der Ausrundung aufsitzt, und der Radius der Anrundung kleiner ist als der der Ausrundung.

20. Behälter nach Anspruch 19, **dadurch gekennzeichnet**, daß zwischen der Deckel-Außenwand (22) und dem an die Oberkante der Seitenwand (5) des Kübels (1) anschließenden, äußeren Oberflächenbereich dieser Seitenwand (5) ein Umfangsspalt gebildet ist, der ein durch einen Innendruck bedingtes Auswölben der Seitenwand (5) ohne Beeinträchtigung der Deckel-Außenwand (22) gestattet.

21. Behälter nach einem der Ansprüche 10 bis 20, **dadurch gekennzeichnet**, daß sich die Außenfläche der Seitenwand (5) des Kübels (1) in dem sich vom Kübel-Außenflansch (17) nach oben erstreckenden Bereich konisch verjüngt, und daß der an den Deckel-Außenflansch (16) angrenzende Innenflächenbereich der Deckel-Außenwand (22) hierzu komplementär ausgebildet ist, um einen dichtenden Kegelsitz zu bilden.

22. Behälter nach einem der Ansprüche 1 bis 10, **dadurch gekennzeichnet**, daß der Kübel (1) und/oder der Deckel (2) und/oder der Ring (3) als einstückiges Spritzgußteil ausgebildet ist.

23. Behälter nach einem der Ansprüche 1 bis 22, **dadurch gekennzeichnet**, daß der Kübel (1) zur Aufnahme vorselektierter Abfälle mindestens eine Unterteilung aufweist.

24. Behälter nach einem der vorhergehenden Ansprüche, **dadurch gekennzeichnet**, daß zwischen dem Außenumfang des Boden-Rohrstutzens (8) und der Unterseite des Bodens (5) ein Kranz von Rohrstutzen-Versteifungsrippen (9) und/oder zwischen der Ringsicke (11) und dem Innen-Ringsteg (12) Deckel-Versteifungsrippen (13) und/oder an dem Ring (3) mit im wesentlichen Y-förmigem Querschnitt kurze (28) und lange (27) Ring-Versteifungsrippen alternierend bis zur Mitte bzw. bis zum Längsbalken des Y und/oder zwischen dem Teil der unteren Fläche des Kübel-Außenflansches (17), der in Eingriff mit der Verschlußanordnung (3) gelangt, um den angrenzenden Teil der Außenfläche der Seitenwand (5) des Kübels (1) Flansch-Versteifungsrippen und/oder zwischen den Unterseiten der Konsolen (24) und der angrenzenden Außenfläche der Seitenwand (5) des Kübels (1) Konsolen-Versteifungsrippen (25) ausgebildet sind.

## Claims

1. Reusable sterilization container, preferably for hospital refuse, having
- a receptacle (1) with a filling opening that is defined by the upper edge of its side wall and is disposed opposite from its bottom, and
- a lid (2) that is for covering the filling opening and is preferably comprised of the same microwave-permeable material as the receptacle and
- a sealing device with a sealing ring (20) between the receptacle (1) and the lid (2),
wherein
- to guarantee a secure and reliable pressure-tight seal between the receptacle (1) and the lid (2), in addition to the sealing ring (20), which is accommodated in grooves (18, 19) in the receptacle (1) and in the lid (2) that are correspondingly embodied in their cross sectional shape,
the sealing device has additional sealing regions (21, 16b; 5b, 22a; 5a, 2a) on the receptacle (1) and the lid (2), and
- a detachable closure device (3, 29) is provided for securing the lid (2) on the receptacle (1) counter to the force of an internal pressure in the receptacle (1), and can be secured in the closed position if need be, and
- the receptacle (1) - and if need be, also the lid (2) - is comprised of a tough plastic that resists stretching, even beyond the boiling temperature of water.

2. Container according to claim 1, characterized in that the side wall (5) of the receptacle (1) tapers all the way from the filling opening to the bottom (4) and can be slid into another receptacle for storage of the receptacles.

3. Container according to claim 3, characterized in that the inner circumference edge is stiffened by means of a circle of bottom-stiffening ribs (7) between the side wall (5) and
bottom (4) of the receptacle (1), whose upper edges form a rest for the outer edge of the bottom (4') of a second receptacle inserted into it, and
the spacing of all of the projections (24, 17) that protrude outward from the outside of the side wall (5) to the upper edge of the side wall (5) is less than the height of the bottom-stiffening ribs (7).

4. Container according to one of claims 2 or 3, characterized in that the outer wall (3) is constituted by a series of truncated cone jackets offset from each other and disposed one above the another.

5. Container according to claim 6, characterized in that a central bottom tubular fitting (8) is embodied on the underside of the bottom (4) bowed toward the inside of the receptacle and does not extend down to the level of the lower edge of the side wall (5) when the receptacle (1) is not acted upon by pressure.

6. Container according to one of claims 1 to 5, characterized in that an upward protruding, preferably continuous outer annular rib (10) is embodied on the top side of the lid (2), which is preferably bowed upward; this annular rib is complementary to bottom edge of the receptacle (1) and receives the bottom edge of the receptacle during storage of closed containers.

7. Container according to one of claims 1 to 6, characterized in that close to the outer circumference of the lid (2), a downward protruding inner annular rib (12) is embodied, which rests fittingly against the inner circumference of side wall (5) of the receptacle (1).

8. Container according to one of claims 1 to 7, characterized in that an annular bead (11) that is indented toward the inside of the container is embodied in the center of the lid (2), wherein the central zone of the non-indented core region of the annular bead (11) is embodied as a flat recess (14) and has a reduced wall thickness in comparison to the other wall thickness of the lid (2).

9. Container according to claim 8, characterized in that an annular groove (15) is disposed in the edge zone of the non-indented core region of the annular bead (11) and is embodied as complementary to the bottom edge of the bottom tubular fitting (8).

10. Container according to one of claims 1 to 9, characterized in that an outer flange (16, 17) is embodied on or near the upper edge of the receptacle (1), as well as on the outer edge of the lid, and that at least one flexible sealing ring (20) is disposed between the outer flanges (16, 17);
for receiving the sealing ring (20) an annular groove (18, 19) constituting an annular channel is embodied in at least one of the outer flanges (16, 17)wherein the height of the annular channel (18, 19) is less than the height of the unstressed sealing ring (20), and the cross sectional surface of the annular channel (18, 19) is at least as great as that of the sealing ring (20).

11. Container according to claim 10, characterized in that outside the annular channel (18, 19), one of the outer flanges (16, 17). preferably the outer receptacle flange (17), comprises an annular projection (21) and the other outer flange, preferably the outer lid flange (16), comprises a recess complementary to the annular projection (21).

12. Container according to one of claims 10 or 11, characterized in that the closure device for securing the lid (2) to the receptacle (1) is embodied as a device (3, 29) for clamping the two outer flanges (16, 17) together.

13. Container according to claim 12, characterized in that the downward pointing surface of the outer receptacle flange (17) and the upward-pointing surface of the outer lid flange (16) converge on each other toward the outside and in particular, comprise a common cross section in the shape of an equal-leg trapezoid, and that a clamping device with at least one clamping element (3) is provided, which has a complementary cross section and can be slid over the two outer flanges (16, 17) from the outside like a clasp.

14. Container according to claim 13, characterized in that the slope angles of the downward-pointing surface of the outer
receptacle flange (17) and the upward-pointing surface of the outer lid flange (16) each exceed the self-locking angle in relation to the sliding direction, of the at least one clamping element (3), preferably in relation to the horizontal direction.

15. Container according to one of claims 13 or 14, characterized in that the clamping element is embodied as an annular body (3) that encompasses both outer flanges (16, 17) from the outside;
said annular body is embodied as a preferably one-piece, resilient ring (3) made of plastic, preferably of the same plastic of which the receptacle (1) and/or the lid (2) is comprised, that the ring (3) has an inner groove (23) pointing toward the center, whose cross section corresponds to that of the two outer flanges (16, 17), that the ring (3) has a radial slot in at least one place, and that the radial slot is bridged by a clamping device (29).

16. Container according to claim 15, characterized in that the clamping device (29) comprises a clamping lever (38), which is supported with its one end on the ring (3) next to the one side of the radial slot so it can pivot around a pivot axis parallel to the ring axis, and which is connected to a point of the ring (3) on the other side of the radial slot via a tension member (36) that is articulatingly coupled to both sides.

17. Container according to one of claims 14 or 15, characterized in that the ring (3) comprises an essentially Y-shaped cross section, wherein the inner flanks of the two arms of Y define the inner groove (23), and that ring-stiffening ribs (27, 28) are embodied between the outer flanks of both arms of the Y and the corresponding side of the longitudinal bar of the Y, respectively.

18. Container according to one of claims 15 to 17, characterized in that for storing the ring (3) when the lid (2) is removed on the outside of the side wall (5) of the receptacle (1), below the outer receptacle flange (17), the annular support is preferably embodied by four brackets (24) distributed around the outer circumference of the side wall (5), wherein the outer circumference described by the brackets (24) is smaller than the outer diameter of the outer flanges (16, 17) and is greater than the inner diameter of the ring (3) when the clamping device (29) is closed, so that the closed ring (3) can loosely, but non-detachably enclose the outer edges of the brackets (24), and the brackets (24) are embodied as handles, preferably because of their disposition sloped slightly downward and outward.

19. Container according to one of claims 7 to 18, characterized in that the side wall (5) of the receptacle (1) is elongated upward beyond the outer receptacle flange (17) and that the lid (2) comprises a sealing groove that opens toward the bottom, between the inner annular rib (12) and its outer lid wall (22) that extends to the outer lid flange (16) and encompasses the upper edge of the receptacle (1); the upper edge of the side wall (5) of the receptacle (1) extends into this sealing groove, wherein preferably the groove bottom of the sealing groove tapers toward the top and ends in a hollow, the upper edge of the side wall (5) of the receptacle (1) comprises a rounded part, which rests on the hollow, and the radius of the rounded part is smaller than that of the hollow.

20. Container according to claim 19, characterized in that a circumference gap is formed between the outer lid wall (22) and the outer surface region of this side wall (5) that adjoins the upper edge of the side wall (5) of the receptacle (1), which gap permits the side wall (5) to bulge because of internal pressure without damaging the outer lid wall (22).

21. Container according to one of claims 10 to 20, characterized in that the outer surface of the side wall (5) of the receptacle (1) tapers conically in the region extending upward from the outer receptacle flange (17), and that the inner surface region of the outer lid wall (22) adjoining the outer lid flange (16) is embodied as complementary to the outer receptacle flange in order to form a sealing conical seat.

22. Container according to one of claims 1 to 21, characterized in that the receptacle (1) and/or the lid (2) and/or the ring (3) is embodied as a one-piece injection-molded part.

23. Container according to one of claims 1 to 22, characterized in that the receptacle (1) has at least one subdivision for receiving preselected refuse.

24. Container according to anyone of the preceding claims, characterized in that a circle of tubular fitting-stiffening ribs (9) is embodied between the outer circumference of the bottom tubular fitting (8) and the underside of the bottom (4). and/or lid-stiffening ribs (13) are embodied between the annular bead (11) and the inner annular rib (12) and/or short and long annular stiffening ribs (27, 28) which have an essentially Y-shaped cross section and extend alternatingly to the center or to the longitudinal bar of the Y, are embodied to the ring (3) and/or flange-stiffening ribs are embodied between the part of the lower surface of the outer receptacle flange (17), which engages with the closure device (3), and the adjoining part of the outer surface of the side wall (5) of the receptacle (1), and/or bracket-stiffening ribs (25) are embodied between the undersides of the brackets (24) and the adjoining outer surface of the side wall (5) of the receptacle (1).

## Revendications

1. Conteneur de stérilisation recyclable, destiné de préférence aux déchets des hôpitaux avec
une poubelle (1) en un matériau laissant traverser les micro-ondes avec une ouverture de remplissage délimitée par le bord supérieur de sa paroi latérale opposée à son fond, couvercle (2) pour recouvrir l'ouverture de remplissage, qui est de préférence réalisée à partir d'un matériau laissant passer les micro-ondes tout comme la poubelle (1), et
un système d'étanchéité avec un anneau d'étanchéité (20) entre la poubelle (1) et le couvercle (2), de sorte que pour garantir une fermeture solide et fiable, étanche à la pression entre la poubelle (1) et le couvercle (2), le système d'étanchéité sauf l'anneau d'étanchéité (20), comportant des gorges (18, 19) de section transversale correspondante et ménagées dans la poubelle (1) et dans le couvercle (2), présente des zones d'étanchéité supplémentaires (21, 16b; 5b, 22a; 5a, 2a) sur la poubelle (1) et sur le couvercle (2) et
un système de fermeture (3, 29) détachable, éventuellement fixable dans la position fermée pour maintenir le couvercle (2) sur la poubelle (1) en opposition à l'action de la pression interne dans la poubelle (1) et
la poubelle (1), éventuellement également le couvercle (2), étant réalisés à partir d'une matière synthétique robuste, pouvant également résister à la dilatation à des températures supérieures à la température d'ébullition de l'eau.

2. Conteneur selon la revendication 1, caractérisé en ce que la paroi latérale (5) de la poubelle (1) va en s'amenuisant à partir globalement de l'ouverture de remplissage jusqu'au fond (4) et peut s'encastrer dans une autre poubelle pour l'empilage des poubelles.

3. Conteneur selon la revendication 1, caractérisé en ce que le bord interne périphérique entre la paroi latérale (5) et le fond (4) de la poubelle (1) est renforcé par une couronne de nervures de renfort de fond (7), dont les bords supérieurs forment un appui pour le bord extérieur du fond (4') d'une deuxième poubelle mise en place et en ce que l'espacement entre toutes les saillies (24, 17) par rapport au bord extérieur de la paroi latérale (5) jusqu'au bord supérieur de la paroi latérale (5) est plus petit que la hauteur des nervures de renfort de fond (7).

4. Conteneur selon l'une des revendications 2 ou 3, caractérisé en ce que la paroi extérieure (5) est formée par une série d'enveloppes tronconiques superposées, décalées entre elles.

5. Conteneur selon l'une des revendications 1 à 4, caractérisé en ce que sur le bord inférieur du fond (5) arqué par rapport au côté intérieur de la poubelle (4) est formée une tubulure de fond médiane (8) qui lorsque la poubelle (1) n'est pas sollicitée par la pression n'arrive pas jusqu'à la hauteur du bord inférieur de la paroi latérale (5).

6. Récipient selon l'une des revendications 1 à 5, caractérisé en ce que sur le côté supérieur du couvercle (2) de préférence arqué vers le haut est formée une entretoise annulaire extérieure (10) faisant saillie vers le haut, de préférence traversante qui est complémentaire au bord inférieur de la poubelle (1) et lors de l'empilage de conteneur fermé reçoit le bord inférieur de la poubelle.

7. Conteneur selon l'une des revendications 1 à 6, caractérisé en ce qu'à proximité de la périphérie extérieure du couvercle (2) est formée une entretoise annulaire interne (12) faisant saillie vers l'intérieur et qui s'appuie de façon correspondante sur la périphérie intérieure de la paroi latérale (5) de la poubelle (1).

8. Conteneur selon l'une des revendications 1 à 7, caractérisé en ce qu'au milieu du couvercle (2) est formée une moulure annulaire (11) dentelée vers le côté interne du conteneur, de sorte que la zone médiane de la région centrale dentelée de la moulure annulaire (11) forme une dépression plate (14) et présente une épaisseur de paroi réduite par rapport à l'épaisseur de paroi restante du couvercle (2).

9. Conteneur selon la revendication 8, caractérisé en ce que dans la zone marginale de la région centrale dentelée de la moulure annulaire (11) est disposée une gorge annulaire (14) qui est formée de façon complémentaire au bord inférieur de la tubulure de fond (8).

10. Conteneur selon l'une des revendications 1 à 9, caractérisé en ce que sur ou à proximité du bord supérieur de la poubelle (1) ainsi que sur le bord extérieur du couvercle est respectivement formé un collet extérieur (16, 17) et en ce qu'entre les collets extérieurs (16, 17) est au moins agencé un anneau d'étanchéité souple (20) pour le logement duquel est prévu dans au moins l'un des collets (16, 17) une gorge annulaire (18, 19) formant un canal annulaire, la hauteur du canal annulaire (18, 19) étant inférieure à la hauteur de l'anneau d'étanchéité non sollicité (20) et la surface transversale du canal annulaire (18, 19) étant au moins aussi grande que celle de l'anneau d'étanchéité (20).

11. Conteneur selon la revendication (10) caractérisé en qu'à l'extérieur du canal annulaire (18, 19) l'un des collets extérieurs (16, 17) de préférence le collet extérieur de poubelle (17) présente une saillie annulaire (21) et l'autre collet extérieur, de préférence le collet extérieur de couvercle (16) présente un évidement complémentaire à la saillie circulaire (21).

12. Conteneur selon l'une des revendications 10 ou 11, caractérisé en ce que le système de fermeture pour le maintien du couvercle (2) sur la poubelle (1) est conçu comme dispositif (3, 29) pour le serrage des deux collets extérieurs (16, 17).

13. Conteneur selon la revendication 12, caractérisé en ce que la surface dirigée vers le bas du collet extérieur de poubelle (17) et la surface dirigée vers le haut du collet extérieur de couvercle (16) convergent entre elles vers l'extérieur et en particulier forment conjointement une section transversale de trapèze équilatère et en ce qu'il est prévu un dispositif de blocage avec au moins un élément de blocage (3) qui présente une section transversale complémentaire et depuis l'extérieur peut coulisser à l'instar d'une broche sur les deux collets extérieurs (16, 17).

14. Conteneur selon la revendication 13, caractérisé en ce que l'angle d'inclinaison de la phase dirigée vers le bas du collet extérieur de poubelle (17) et de la phase dirigée vers le haut du collet extérieur de couvercle (16) surmontent respectivement en opposition à la direction de coulissement d'au moins un élément de serrage (3), de préférence par rapport à l'angle d'auto-blocage horizontal.

15. Conteneur selon la revendication 13 ou 14, caractérisé en ce que l'élément de blocage est conçu sous forme d'un corps annulaire (3) enserrant les deux collets extérieurs (16, 17) depuis l'extérieur qui, de préférence, se présentent sous forme d'un anneau élastique d'une seule pièce (3) en matière synthétique, de préférence de la même matière synthétique que la poubelle (1) et/ou le couvercle (2) et en ce que l'anneau (3) présente une gorge interne (23) dirigée vers le milieu dont la section transversale correspond à celle des deux collets extérieurs (16, 17), en ce que l'anneau (3) présente sur au moins un emplacement une fente radiale et en ce que la fente radiale est reliée par un dispositif tendeur (29).

16. Conteneur selon la revendication 15, caractérisé en ce que le dispositif tendeur (29) présente un levier tendeur (38) qui est logé avec une de ses extrémités sur l'anneau (13) à proximité d'un côté de la fente radiale de façon basculante par rapport à l'axe basculant parallèlement à l'axe de l'anneau et qui est raccordé par un élément de traction (36) accouplé de façon articulée des deux côtés avec une position de l'anneau (3) sur l'autre côté de la fente radiale.

17. Conteneur selon l'une des revendications 14 ou 15, caractérisé en ce que l'anneau (3) présente une section transversale sensiblement en forme de Y, les flans internes des deux branches de l'Y délimitant la gorge interne (23) en ce qu'entre eux les flans externes des deux branches de l'Y et le côté correspondant de la partie longitudinale de l'Y sont respectivement formés des nervures de renfort annulaire (27, 28).

18. Conteneur selon l'une des revendications 15 à 17, caractérisé en ce que pour l'application de l'anneau (3) lorsque le couvercle (2) est enlevé sur le côte latéral de la paroi latérale de la poubelle (1) au-dessous du collet externe de poubelle (17), il est formé un appui annulaire (24) de préférence constitué de quatre consoles réparties sur la périphérie extérieure de la paroi latérale (5), la périphérie extérieure décrite par les consoles (42) étant plus petites que le diamètre extérieur des brides externes (16, 17) et plus grandes que le diamètre interne de l'anneau (3) avec le dispositif tendeur fermé (29), de sorte que l'anneau fermé (3) renferme les bords externes des consoles (24) de façon libre mais inamovible et les consoles (24) sont formées de préférence par leur disposition inclinée légèrement vers le bas et vers l'extérieur comme poignée.

19. Conteneur selon l'une des revendications 16 à 18, caractérisé en ce que la paroi latérale (5) de la poubelle (1) est prolongé par le collet externe de poubelle (17) vers le haut et en ce que le couvercle (2) entre l'entretoise annulaire interne (12) et sa paroi externe de couvercle (22) s'étendant jusqu'au collet externe de couvercle (16) et renfermant par l'extérieur le bord supérieur de la poubelle (1) présente une gorge d'étanchéité ouverte vers le bas dans laquelle s'étend le bord supérieur de la paroi latérale (5) de la poubelle (1), de sorte que, de préférence, la base de la gorge d'étanchéité va en s'amincissant vers le haut et se termine par un congé, le bord supérieur de la paroi latérale (5) de la poubelle (1) présente un arrondi qui repose sur le congé et le rayon de l'arrondi est inférieur à celui du congé.

20. Conteneur selon la revendication 19 caractérisé en ce qu'entre la paroi externe de couvercle (22) et la zone de surface extérieure se raccordant sur le bord supérieur de la paroi latérale (5) de la poubelle (1) de cette paroi latérale (5) est formé un espace périphérique qui permet une voussure conditionnée par la pression interne de la paroi latérale (5) sans porter atteinte à la paroi extérieure de couvercle (22).

21. Conteneur selon l'une des revendications 10 à 20, caractérisé en ce que la surface externe de la paroi latérale (5) de la poubelle (1) va en s'amincissant dans la zone s'étendant vers le haut à partir du collet externe de poubelle (17) de façon conique et en ce que la zone de surface interne limitrophe du collet externe de couvercle (16) de la paroi externe de couvercle (22) est formé de façon complémentaire de façon à constituer un siège conique d'étanchéité.

22. Conteneur selon l'une des revendications 1 à 10, caractérisé en ce que la poubelle (1) et/ou le couvercle (2) et/ou l'anneau (3) sont conçus comme éléments de moulage par pulvérisation d'une seule pièce.

23. Conteneur selon l'une des revendications 1 à 22, caractérisé en ce que la poubelle (1) présente au moins une subdivision destinée à recevoir des déchets présélectionnés.

24. Conteneur selon l'une des revendications précédentes, caractérisé en ce qu'entre la périphérie externe de la tubulure de fond (8) et le côté inférieur du fond (5) est prévue une couronne de nervures de renforcement de tubulure (9) et/ou entre la moulure annulaire (11) et l'entretoise annulaire interne (12) sont prévues des nervures de renfort du couvercle (13) et/ou sur l'anneau (3) avec des nervures de renfort annulaires courtes (28) et longues (27) en section transversale sensiblement en forme de Y et alternent jusqu'à la partie médiane ou jusqu'à la partie longitudinale du Y et/ou entre la partie de la surface inférieure du collet externe de poubelle (17) qui vient en engagement avec le système de fermeture (3) autour de la partie limitrophe de la surface externe de la paroi latérale (5) de la poubelle (1) des nervures de renforcement de collet et/ou entre le côté inférieur des consoles (24) et la surface externe limitrophe de la paroi latérale (5) de la poubelle (1) sont formées des nervures de renfort de console (25).
